(19) **Europäisches Patentamt / European Patent Office / Office européen des brevets**

(11) **EP 2 471 513 B1**

(12) **EUROPEAN PATENT SPECIFICATION**

(45) Date of publication and mention of the grant of the patent:
**10.06.2015 Bulletin 2015/24**

(51) Int Cl.:
*A61K 9/14* (2006.01)   *A61K 31/00* (2006.01)
*A61K 31/192* (2006.01)   *A61K 9/16* (2006.01)

(21) Application number: **10016118.1**

(22) Date of filing: **28.12.2010**

(54) **Process for producing an active pharmaceutical ingredient (API) preparation in the form of beads**

Verfahren zur Herstellung einer Zubereitung mit aktivem pharmazeutischem Inhaltsstoff in Form von Kügelchen

Procédé de production d'ingrédients pharmaceutique actif et préparation sous la forme de billes

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**04.07.2012 Bulletin 2012/27**

(73) Proprietor: **LEK Pharmaceuticals d.d.**
**1526 Ljubljana (SI)**

(72) Inventors:
• **Homar, Miha**
**1526 Ljubljana (SI)**

• **HORVAT Matej**
**1526 Ljubljana (SI)**
• **CEGNAR Mateja**
**1526 Ljubljana (SI)**
• **KERC Janez**
**1526 Ljubljana (SI)**

(74) Representative: **Prüfer & Partner GbR**
**European Patent Attorneys**
**Sohnckestraße 12**
**81479 München (DE)**

(56) References cited:
**EP-A1- 1 338 274    EP-A1- 1 498 116**
**WO-A1-99/30687    WO-A2-02/094223**

## Description

<u>Field of the Invention</u>

The present invention relates to the field of pharmaceuticals, and in particular to a process for producing an active pharmaceutical ingredient (API) preparation in the form of beads. Furthermore, the present invention relates to an API preparation in the form of beads and a pharmaceutical composition comprising said API preparation.

<u>Background of the invention</u>

Conventionally, active pharmaceutical ingredient (API) preparations in the form of beads are prepared from original API particles which are formed into beads of larger size than the original API particles, wherein size enlargement is carried out by e.g. extrusion-spheronization, hot-melt extrusion, freeze pelletization, cryopelletisation, wherein an overview of these processes is given e.g. in A. Rahman et. al., "Recent Advances in Pelletization Technique for Oral Drug Delivery: A Review in Current Drug Delivery", 2009, 6, 122-129. Further processes for size enlargement such as e.g. spherical agglomeration and pelletisation by a fluid bed system are described in L. Prabhakaran et. al., " Pharmaceutical Micropellets : An Overview", Latest Reviews, 2009, Vol. 7, Issue 4, published on the website www.pharmainfo.net.

WO 02/094223 A2, EP 1 338 274 A1 and WO 99/30687 respectively disclose conventional wet ball milling processes which are applied in order to reduce the particle size of API particles. After ball milling, the size reduced API particles are spray dried in order to obtain a dried powder which particles have a size of one micron or less.

These aforementioned processes have limitations, for example in terms of non-uniformity of the obtained API preparations in the form of beads, in view of the bead size and in view of the relatively low drug content of said beads.

In general, the conventional processes are laborious and require complex production apparatuses, complex process control and/or multiple process steps. For example, in order to obtain an API preparation in the form of beads having a controlled release dissolution profile, typically e.g. a subsequent layering/coating process is applied in order to provide a controlled API release profile.

The object of the present invention is to provide an improved process for producing API preparations in the form of beads, to provide an improved API preparation in the form of beads and to provide an improved pharmaceutical composition comprising said API preparation in the form of beads.

## Summary of the invention

The object is solved by a process for producing an API preparation in the form of beads according to claim 1 and by an API preparation in the form of beads according to claim 8. The present invention further provides a pharmaceutical composition according to claim 10. Preferred embodiments are set forth below and in the subclaims.

Various aspects, advantageous features and preferred embodiments of the present invention as summarized in the following items, respectively alone or in combination, contribute to solving the object of the invention:

> (1) A process for producing an active pharmaceutical ingredient (API) preparation in the form beads, wherein the process comprises:
>
>> a) providing an aqueous suspension comprising an API in the form of undissolved particles, a stabilizing agent, an inorganic salt and water,
>> b) subjecting an aqueous suspension defined in a) to ball milling, wherein the average original particle size of the API provided in a) is increased by aggregation, the aggregated API preparation in the form of beads having an average diameter of at least 0.1 mm, preferably 0.2 to 5 mm, wherein the average original particle size of the API provided in a) is 5 $\mu$m to 1 mm, and wherein in b) the revolution speed of ball milling is set within a range of 300 to 500 min$^{-1}$.

The term "pharmaceutically active ingredient (API)" as used herein means any active pharmaceutical ingredient intended for treatment or prophylaxis of a disease of a mammal. In general, it means any active pharmaceutical ingredient that has an effect on the physiological conditions of a mammal.

The term "suspension" as used herein means a heterogeneous mixture of a fluid phase and solid particles, wherein the solid particles are dispersed throughout the fluid phase.

The term "stabilizing agent" as used herein means is a substance or mixture of substances which inhibits separation of fluid phase(s) and solid phase(s) comprised in a suspension as defined above.

The term "ball milling" as used herein means a process wherein shear forces are applied to a starting material by means of so-called milling balls located in a milling vessel. Typically and preferably, the milling vessel is rotated, wherein the milling balls collide with each other and with the API particles provided as the starting material.

The term "original particle size" as used herein means the particle size of the API particle provided as the starting material in a) of item (1).

In the following, the term "active pharmaceutical ingredient (API) preparation in the form beads" may be abbreviated by the term "API bead(s)".

According to this aspect of the invention, it was surprisingly found that a ball milling process enables a simple and effective process for preparing highly desirable API beads. In particular, the present process provides for excellent operability which limits the risk of product quality limitations due to operating errors, while still providing API beads having excellent physical and chemical properties. In particular, the dissolution profile of said API beads can be advantageously controlled by appropriately selecting the composition of a suspension used as the starting material and/or appropriately setting the ball milling parameters, wherein formation of the API bead and setting dissolution characteristics can be done in a single wet ball milling process - no further/subsequent process steps being necessary.

(2) The process according to item (1), wherein a ratio of average original particle size of the API provided in a) to diameter of the API beads obtained in b) is 1/1000 to 1/10, preferably 2/1000 to 8/100.

(3) The process according to item (1) or (2), wherein the API provided in a) *per se* has a solubility in water of less than 1 g/l, preferably less or equal to 0.6 g/l, more preferably less or equal to 0.1 mg/ml, and in particular less or equal to 0.01 mg/ml.

The term "solubility" as used herein means a solubility as defined in the US or European Pharmacopoeia, which can e.g. be determined according to the U.S. Pharmacopeia (U.S.P.) XXI, page 1441.

(4) The process according to any one of items (1) to (3), wherein in the aqueous suspension provided in a) of claim 1, the weight ratio of amount of API to amount of water (API/water) is up to 1/1, preferably 0.1/1 to 0.8/1 and more preferably 0.15/1 to 0.7/1.

(5) The process according to any one of items (1) to (4), wherein the total amount of API is 8 to 45 % by weight relative to total weight of the aqueous suspension provided in a) of claim 1, preferably 10 to 40 % by weight.

According to this embodiment, in a) of item (1), a suspension is provided having a composition which is particularly suitable for the subsequent ball milling in b) of item (1), since the amount of API within the aqueous suspension is within a range wherein dispersion of the solid API phase in the fluid/aqueous phase is provided, which is advantageous in that said suspension can be wet milled easily. An API load higher than that defined in item (5) may tend to circumstances that the fluid phase in form of aqueous suspension is dissolved in or absorbed by the solid API phase. In this case, a paste-like mass may form which - depending on the characteristics of the API and the milling process parameters - may have a negative impact on the milling process and/or on the formation of API beads having an increased size.

(6) The process according to any one of items (1) to (5), wherein the average original particle size of the API provided in a) is 5 $\mu$m to 1 mm, preferably 6 $\mu$m to 0.5 mm, more preferably 7 $\mu$m to 250 $\mu$m and in particular 8 $\mu$m to 125 $\mu$m.

(7) The process according to any one of items (1) to (6), wherein the API preparation in the form of beads formed in b) have a diameter of 0.4 to 3 mm, preferably 0.5 to 2 mm.

(8) The process according to any one of items (1) to (7), wherein ball milling in b) is conducted at a loading with milling beads at a ratio of bulk volume of milling beads to volume of milling vessel of 0.2/1 to 0.75/1, preferably 0.22/1 to 0.6/1.

The term "bulk volume" as used herein means the volume of a close-packing/packing of spheres in form of milling beads.

(9) The process according to any one of items (1) to (8), wherein the density of milling balls is 3 to 17 kg/dm$^3$, preferably 3.5 to 10 kg/dm$^3$ and more preferably 5 to 8.5 kg/dm$^3$

(10) The process according to any one of items (1) to (9), wherein milling balls have a diameter of 0,05 to 5 mm, preferably 0.4 to 2 mm, more preferably 0.6 to 0.8 mm.

(11) The process according to any one of items (1) to (10), wherein the volume ratio of milling beads to the aqueous suspension provided in a) is 0.9/1 to 2/1, preferably 1/1 to 1.6/1.

(12) The process according to any one of items (1) to (11), wherein the stabilizing agent is a wetting agent, preferably selected from the group consisting of polyoxyethylene sorbitan esters, polyoxyethylene alkylphenyl ethers, polyoxamers, polyoxyethylene fatty acid esters, polyoxyethylene alkyl ethers, polyoxyethylene fatty acid esters, propylene glycol fatty acid esters, fatty acids and salts thereof, glyceryl fatty acid esters, sorbitan esters, quaternary ammonium compounds, alkyl sulfates and their salts, other ionic surfactants (e,g, bile salts), mono- and diglycerides, sterol and sterol derivatives, unsaturated polyglycosed glycerides such as e.g. apricot kernel oil PEG-6 esters (commercially available e.g. under the brand name Labrafil®), polyoxyglycerides such as e.g. PEG-8 capryllc glycerides (commercially available e.g. under the brand name Labrasol®) and mixtures thereof, more preferably the wetting agent is a polyoxyethylene sorbitan ester, even more preferably polysorbat 20-120, and in particular polysorbat 80.

The term "wetting agent" as used herein means a substance or mixture of substances which reduces the surface tension of a liquid, wherein spreading of the liquid on a solid surface or penetration of a liquid into a solid phase is improved. That is, the wetting agent provides for a more intimate contact of a fluid phase and a solid phase.

According to this beneficial embodiment of the invention, stabilizing agents are selected which provide for a particularly advantageous suspension for subsequent ball milling in step b) of item (1). It is believed that the above mentioned wetting agents not only provide for the necessary wetting of the original API particles for wet ball milling, but also have a positive impact on the formation of API beads owing to their surface active properties.

(13) The process according to one of items (1) to (12), wherein the stabilizing agent is a suspending agent, preferably selected from the group consisting of cellulose derivatives, alginates, chitosan, dextrans, gelatin, polyethylene glycols, polyoxyethylene- and polyoxypropylene ethers, polyvinylpyrrolidone, polyvinyl alcohol, and mixtures thereof, preferably a cellulose derivative selected from the group consisting of methyl cellulose, sodium carboxymethyl cellulose and hydroxypropyl methyl cellulose.

The term "suspending agent" as used herein means a substance or mixture of substances providing for improved dispersion of a solid phase in an external fluid phase. Preferably, a solid phase in form of API particles provided in a) of item (1) is essentially uniform dispended within the fluid phase.

(14) The process according to item (12) or (13), wherein said wetting agent(s) and said suspending agent(s) are used alone or in combination with each other as the stabilizing agent, preferably the total amount of wetting agent(s) is 0.1 to 15 % by weight relative to total weight of the aqueous suspension provided in a) and/or the total amount of suspending agent(s) is 0.1 to 15 % by weight relative to total weight of the aqueous suspension provided in a), more preferably the total amount of wetting agent(s) is 0.8 to 10 % by weight relative to total weight of the aqueous suspension provided in a) and/or the total amount of suspending agent(s) is 0.8 to 10 % by weight relative to total weight of the aqueous suspension provided in a), and in particular the stabilizing agent is a wetting agent in the total amount of 1 to 6 % by weight relative to total weight of the aqueous suspension provided in a).

(15) The process according to any one of items (1) to (14), wherein the inorganic salt is selected from the group consisting of sodium, potassium, magnesium and calcium salts of either monoatomic or polyatomic counter ions, preferably the inorganic salt is sodium chloride.

In this way, without wishing to be bound to theory, the charge density within the dispersion provided in a) of item (1) is suitably adjusted wherein a particularly advantageous $\zeta$ (Zeta)-potential is adjusted in the suspension. Zeta potential means the electrical potential which is generated by accumulation of ions at the surface of a colloidal particle. It is believed that this advantageous Zeta-potential has a positive impact on the formation of API beads during the wet ball milling process In b) of item (1).

(16) The process according to any one of items (1) to (15), wherein the total amount of inorganic salt(s) is 1 to 30 % by weight relative to total weight of the aqueous suspension provided in a), preferably 4 to 25 % by weight and more preferably 8 to 22 % by weight.

(17) The process according to any one of items (1) to (16), wherein the aqueous suspension provided in a) further comprises auxiliary agent(s) or pharmaceutically acceptable excipients, preferably selected from the groups consisting of buffering agents, defoaming agents and preservatives.

The term "auxiliary agent" as used herein means a substance or mixture of substances which do(es) not exert a biologically relevant effect of its/their own, but provides for e.g. improved formulation of a pharmaceutical preparation, supports effects of a pharmaceutically active ingredient(s) and/or improves appearance of the pharmaceutical composition. When auxiliary agents are used, their choice will depend on the API and on the process conditions.

The term "buffering agent" as used herein means a substance or mixture of substances providing for adjustment of the pH value of a liquid to a certain pH range and to prevent change of pH to the outside of this range.

The term "defoaming agent" as used herein means a substance or mixture of substances which reduce the generation of foam, in particular during a preparation process.

The term "preservatives" as used herein means a substance or mixture of substances which prevents decomposition of a pharmaceutical composition, e.g. microbial or bacterial decomposition.

In principle and advantageously, the present process can dispense with auxiliary agents, since there is generally no problem such as e.g. foam formation or negative impact of pH. However, depending on the characteristics of the API provided in step a) of item (1), such auxiliary agent(s) may be useful. A buffering agent may also be advantageous for the pharmaceutical application of the API bead. Preservative(s) may be predominantly added in order to prolong the shelf-life of the obtained API bead, wherein such preservatives should be suitably selected with the proviso that the preservative has no negative impact on the formation of API beads during ball milling.

Further, conventionally and typically, pharmaceutical acceptable excipients may be used, although the process of the present invention may beneficially dispense with such excipient addition, if desired. As further alternative, if desired, any pharmaceutically acceptable excipient may be added externally, or mixed with the API beads after they have been produced according to the process.

(18) The process according to any one of items (1) to (17), wherein the total amount of auxiliary agent(s) is 0 to 10 % by weight relative to total weight of the aqueous suspension provided in a), preferably 0.01 to 5 % by weight.

(19) The process according to any one of items (1) to (18), wherein the API provided in a) has at least one characteristic selected from the group consisting of:

- at least one polar group is present in the API molecule, preferably a keto and/or carboxylic acid group, more preferably a keto and a carboxylic acid group;
- a molecular weight of 150 to 1000 g/mol, preferably 180 to 500 g/mol, more preferably 220 to 400 g/mol and in particular 240 to 300 g/mol;
- a melting point of 60-150 °C, preferably 80-110 °C, more preferably 90-100°C.

Preferably, the API as defined in item (19) belongs to the class of APIs to be orally administered. A suitable API class includes for example, without being limited thereto, non-oploics, that is natural or synthetic substances being neither a opioid compound nor an opiate, and thus, a non-opioic does not bind to specific opioid receptors in the nervous system and does not have agonist or antagonist effects at these receptors.

(20) The process according to any one of items (1) to (19), wherein the beads obtained from the ball milling are substantially spherical, preferably having a sphericity of at least 0.5, more preferably 0.7, and in particular 0.8.

The term "substantially spherical" as used herein means that the beads posses a regularly shaped, rounded form resembling a sphere.
The term "spherical/sphericity" as used herein is defined as follows:

$$S = (4 \cdot \pi \cdot A) / (P^2 \cdot A)$$

, wherein S is the sphericity, A denotes the surface area-value of a particle and P denotes the circumference of a particle. A sphericity value S of 1 means that the particle is completely spherical. Advantageously, S is determined based on microscopic pictures and/or subsequent computational picture analysis by suitable software.
According to this embodiment of the invention, the process provides for API beads having useful shape and a proper appearance. Thereby, the API beads are uniform and when e.g. filled in transparent capsules, the API beads should have a proper appearance.

(21) The process according to any one of items (1) to (20), wherein the beads obtained from the ball milling have a porosity of 1 to 30%, preferably 5 to 20%, more preferably 7 to 15% and in particular 8 to 12%.

The term "porosity" as used herein means the porosity within the API bead which is defined as follows:

$$\varepsilon_g\ [\%] = (1 - \rho_g/\rho_s) \cdot 100$$

, wherein $\varepsilon_g$ Is the porosity given as percentage value, $\rho_g$ denotes the agglomerate density (related by the volume occupied by the agglomerates including internal porosity) and $\rho_s$ denotes the true density of the solid (calculated by the mass of the solid divided by its volume excluding open or closed pores).
In this way, it is possible to obtain different intra-agglomerate porosities suitably adapted for different applications. Without wishing to be bound to theory, it is believed that said porosity enables an advantageous incorporation of fluid into the bead by capillary action due to the porous structure of the bead. Thereby, dissolution of the API bead may be significantly improved.

(22) The process according to any one of items (1) to (21), wherein in b), the revolution speed of ball milling is set within a range of 300 to 500 min$^{-1}$.

(23) The process according to any one of items (1) to (22), wherein in b), the milling time is set within a range of 20 to 400 min, preferably 30 to 360, more preferably 100 to 250.

(24) The process according to any one of items (1) to (23), wherein In b), the volume ratio of total load volume of aqueous suspension of a) to volume of the milling vessel is in a range of 0.2/1 to 0.6/1, preferably 0.22/1 to 0.5/1.

(25) The process according to any one of items (1) to (24), wherein the milling ball material has a Mohs hardness of 5 to 10, preferably 6 to 9.5 and more preferably 8 to 9.

(26) The process according to any one of items (1) to (25), wherein the milling ball material is selected from the group consisting of hard porcelain, steatite, $Al_2O_3$, $ZrO_2$, steel, hard metal, preferably the milling ball material is selected from the group consisting of $Al_2O_3$, $ZrO_2$, steel, more preferably the milling ball material is $ZrO_2$.

(27) The process according to item (26), wherein the surface of the milling ball is coated or uncoated, preferably uncoated.

(28) An active pharmaceutical ingredient (API) preparation in the form of beads based on an API having a solubility in water of less than 1 g/l, optionally a stabilizing agent and optionally an inorganic salt, wherein:

(i) the beads of the API preparation are formed by ball milling particulate API to obtain an API preparation in the form of beads having an average diameter of at least 0.1 mm, preferably 0.2 to 5 mm; preferably, ball milling is applied by a process according to any one of items (1) to (27);
(ii) API primary particles have an average diameter of less than 20 $\mu$m, yet even more preferably less than 10 $\mu$m and in particular less than 5 $\mu$m, wherein the bead porosity is 1 to 30%, and wherein the API preparation is in the form of spherical beads having a sphericity of at least 0.5.

The term "primary particle" as used herein means the single API particles comprised in the API preparation in the form of beads. These primary particles may preferably have a smaller particle size compared to the original particle size of API particles provided in a) of item (1), owing to the milling process.
Besides of the aforementioned primary particles, the API preparation in the form of beads may also comprise "secondary particles", that is agglomerates of said primary particles within the API bead, wherein formation of such secondary particles is further associated with the milling process. In case API secondary particles are desired in the API bead, they preferably have an average diameter of less than 400 $\mu$m, preferably less than 280 $\mu$m, more preferably less than 150 $\mu$m, even more preferably less than 80 $\mu$m and in particular less than 30 $\mu$m.
Since the API preparation in the form of beads is obtained by ball milling, preferably by ball milling according to any one of items (1) to (27), a stabilizing agent and/or an inorganic salt used in said ball milling process may be identified in the API beads in minor amounts, that is, the API preparation in the form of beads may be based on a mixture comprising an API having a solubility in water of less than 1 g/l, a stabilizing agent and an inorganic salt.
According to this beneficial aspect of the invention, an advantageous API preparation in the form of beads is provided having both a pleasant appearance owing to its bead form and an advantageous dissolution characteristics owing to the advantageous API's primary particle size.

(29) The API preparation according to item (28), wherein the porosity is 1 to 30%, preferably 5 to 20%, more preferably 7 to 15% and in particular 8 to 12%.

As to this embodiment of the API preparation and its significance, reference is made to the explanations in item (21) above.

(30) The API preparation according to item (28) or (29), wherein the API preparation in the form of beads comprises the API in an amount of at least 90% by weight relative to the weight of the composition in the form of beads, preferably 93.00-99.99% by weight, more preferably 96.00-99.90% by weight.

According to this beneficial embodiment of the invention, API beads having an advantageous high API content are provided, wherein a content of excipients can be significantly reduced or it can be even dispensed with excipients.
Preferably, the API preparation in the form of beads according to the present invention is free of or at least essentially free of excipients, more preferably free of excipients, and in particular free of a binder, a disintegrant and/or a bulk polymer.
The term "binder" as used herein means a binding agent which improves adhesion in between API particles.
The term "disintegrant" as used herein means an agent providing for rapid disintegration of a pharmaceutical composition into smaller fragments when in contact with water, wherein dissolution of the pharmaceutical composition and in particular of an API comprised therein is improved.
The term "bulk polymer" as used herein means a polymeric filling agent which is typically added to a pharmaceutical composition in large amounts, at least in an amount larger than 6% by weight relative to the total weight of the pharmaceutical composition.

(31) The API preparation according to any one of items (28) to (30), wherein the API preparation In the form of beads is prepared by a ball milling process, preferably a ball milling process according to any one of items (1) to (18).

(32) The API preparation according to any one of items (28) to (31), wherein the API comprised in said bead has characteristics selected from the group consisting of:

- at least one polar group is present in the API molecule, preferably a keto and/or carboxylic acid group, more preferably a keto and a carboxylic acid group;
- a molecular weight of 150 to 1000 g/mol, preferably 180 to 500 g/mol, more preferably 220 to 400 g/mol and in particular 240 to 300 g/mol;
- a melting point of 60-150 °C, preferably 80-110 °C, more preferably 90-100°C;
- a solubility in water less or equal to 0.6 g/l, more preferably less or equal to 0.1 mg/ml, and in particular less or equal to 0.01 mg/ml.

Preferably, the API as defined in item (32) belongs to the class of APIs to be orally administered. More preferably, the API is selected from the group consisting of tadalafil, fenofibrate, cholecalciferol (vitamin D), simvastatin, ezetimibe, ketoprofen, celecoxib, candesartan, atorvastatin, sirolimus, non-steroidal anti-inflammatory drugs (NSAIDs) such as naproxen and ibuprofen, megestrol acetate, ritonavir (HIV protease inhibitor), or ciclosporin; even more preferably, the API is selected from the group consisting of fenofibrate, cholecalciferol (vitamin D), simvastatin, ezetimibe, ketoprofen, celecoxib, candesartan, atorvastatin, and sirolimus; and in particular, the API is ketoprofen (2-(3-benzoylphenyl)-propionic acid).

(33) The API preparation according to any one of items (28) to (32), wherein the API preparation in the form of beads is substantially spherical, preferably having a sphericity above 0.5, preferably 0.7 more preferably 0.8.

As to this embodiment of the API preparation and its significance, reference is made to the explanations in item (20) above.

(34) A pharmaceutical composition comprising the API preparation according to any one of items (28) to (33) or consisting only of the API preparation according to any one of items (28) to (33).

This aspect of the invention provides pharmaceutical compositions which are obtained from the present API beads. Hence, these pharmaceutical compositions have improved physical and/or chemical properties owing to the incorporated API beads.

(35) The pharmaceutical composition according to item (34), wherein the composition is in a form selected from pellets, capsules, granules or compressed tablets, and/or the composition is in a form filled in sachets or in capsules, preferably the pharmaceutical composition is in form of a capsule filled with pellets.

The term "pellet" as used herein means that the API composition in the form of beads prepared by the process according to the invention is directly used as a dosage form.

The term "compressed tablets" as used herein means a solid product formed by compression of a pharmaceutical composition such that the composition remains in an unitary form for delivery.

**Detailed description of the invention**

The present invention is now described in more detail by referring to further preferred and further advantageous embodiments and examples, which are however presented for illustrative purposes only and shall not be understood as limiting the scope of the present invention.

Conventionally, the dissolution rate of active pharmaceutical ingredients (API) having a poor solubility in aqueous media is tried to be enhanced by applying such APIs to a grinding process in an attempt to increase the solubility of the API. According to such a conventional concept, API particle size shall be reduced to $\mu$m or nm scale. Increase the surface of the API shall favour its solubility and hence improve bioavailability. Different techniques have been used for decreasing the particle size down to micro- or nanosize range, such as e.g. wet milling, stirred media milling, cryo milling, high pressure homogenization, etc.

Surprisingly, when providing for size enlargement of surprisingly particulate API having a solubility in water of less than 1 g/l during a ball mill process of an API dispersion in the presence of a stabilizing agent and an inorganic salt, an API preparation in the form of beads with strongly agglomerated API particles in the form of beads larger than 0.1 mm, particularly in the range of 0.2 to 5 mm, were formed a substantially increased size compared to the originally applied API particle starting material, leading to beneficial characteristics associated with the enlargement through the ball milling process. That is, an unexpected particle size increase took place, wherein the obtained API preparation in the form of beads had highly desirable characteristics.

Therefore, the present process for preparing an API preparation is highly advantageous, since conventional processes for producing API preparations in the form of beads, such as e.g. extrusion-spheronization, hot-melt extrusion, freeze pelletization, cryopelletisation, agglomeration in a drug-solvent/bad solvent pouring system, or pelletisation by a fluid bed system, are limited e.g. in terms of non-uniformity of the obtained API preparation in the form of beads, in view of the bead size and in view of the relatively low API content of such beads.

Furthermore, the prior art processes for preparing an API preparation in the form of beads are laborious and in general require complex production apparatuses, complex process control and/or multiple process steps. Mostly, a layering/coating process is necessary subsequent to a conventional process for preparing API beads in order to provide API beads with a controlled API release profile. Alternatively or additionally, functional additives are incorporated into API beads in order to improve dissolution characteristics, wherein the testing of the compatibility of such additives to the API and further excipients present in the preparation requires elaborate screening tests.

In contrast to the aforementioned conventional processes for preparing an API preparation in the form of beads, the present invention provides a simple and effective process for preparing API beads having improved characteristics such as e.g. an advantageous dissolution profile and/or a surprisingly high API content within the bead. In general, the present process provides for excellent operability which limits the risk of product quality limitations due to operating errors, while still providing API beads having excellent physical and chemical properties. In particular, the dissolution profile of the API beads obtained can be advantageously controlled by appropriately selecting the composition of a suspension used as the starting material and/or appropriately setting the ball milling parameters. In this connection, it is noteworthy that formation of the API beads and the setting of dissolution characteristics is accomplished in one single process. Further advantageously and if desired, the improved combination of preparations can be accomplished even without using any binder, or without disintegrant, or without functional polymer, or without any of these excipients in the API beads themselves.

The API preparation in the form of beads prepared by the present process offers particular therapeutic advantages owing to its enhanced dissolution characteristics. For example, when taken up in the gastrointestinal tract (GI), the API preparation of the present invention provides for less gastric irritation by limiting localized buildup and dose dumping in the GI.

Furthermore, the present API preparation provides for many technical advantages such as good flowability owing to its uniform size and spherical shape, high physical integrity of its spherical agglomerates, high strength, low friability, narrow particle size distribution, superior surface quality for coating applications and uniform packing characteristics.

It is further believed that size enlargement by API particle agglomeration through ball milling leads to a particular API bead texture beneficially characterized by an intra-agglomerate porosity, i.e. porosity present within the API beads, wherein porosity is measurable e.g. by quantitative mercury porosimetry quantitative determination by using optical microscopy and scanning electron microscopy (SEM) together with image analysis, preferably by last-mentioned microscopical image analysis as described e.g. by M. Salako et. al ., "Investigations into the deformity and tensile strength of pellets, Int. J. Pharm., 1998, 168, 49-57. Said porosity is apparently associated with a strong micro-capillary effect which in turn strongly favors dissolution of the API compound in aqueous media despite of its poor solubility as such.

The following examples are merely illustrative of the present invention and they should not be considered as limiting the scope of the invention in any way. The examples and modifications or other equivalents thereof will become apparent to those versed in the art in the light of the present entire disclosure. In particular, the principles of the present invention will be illustrated and demonstrated by an exemplified use of ketoprofen as API, but the technical concepts or the present invention as disclosed herein can be applied correspondingly to other APIs contemplated herein as well.

Brief description of the Figures

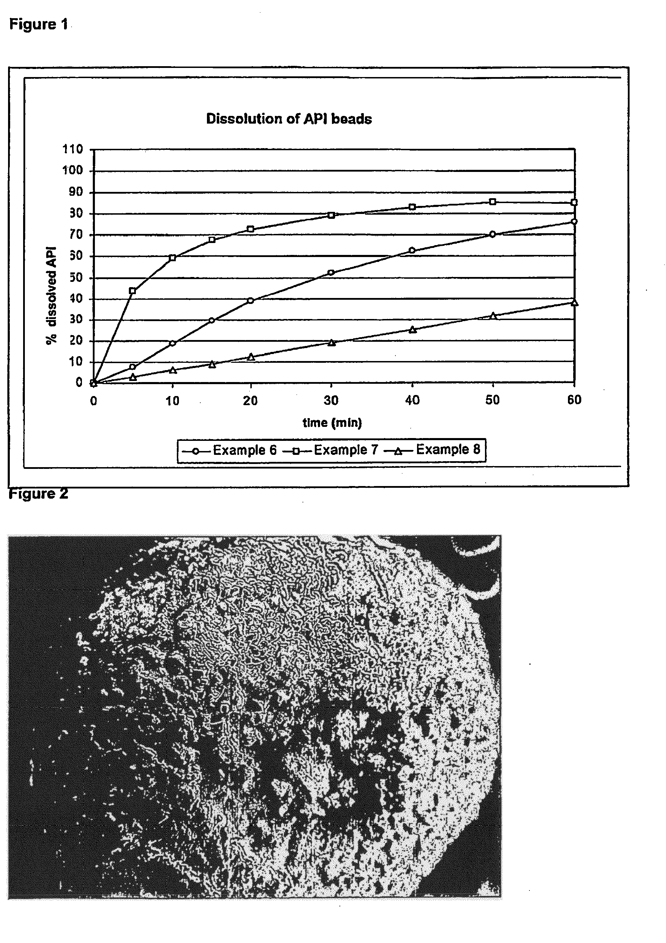

Figure 1 is a dissolution profile diagram of API beads prepared according to Example 6, 7 and 8 according to the invention. In Example 9, dissolution testing conditions are given and the results thereof in form of the diagram depicted in Fig. 1 is discussed.

Figure 2 depicts an API composition in the form of a bead prepared according to Example 7, microscopically magnified by factor 50. This bead has a substantially spherical shape.

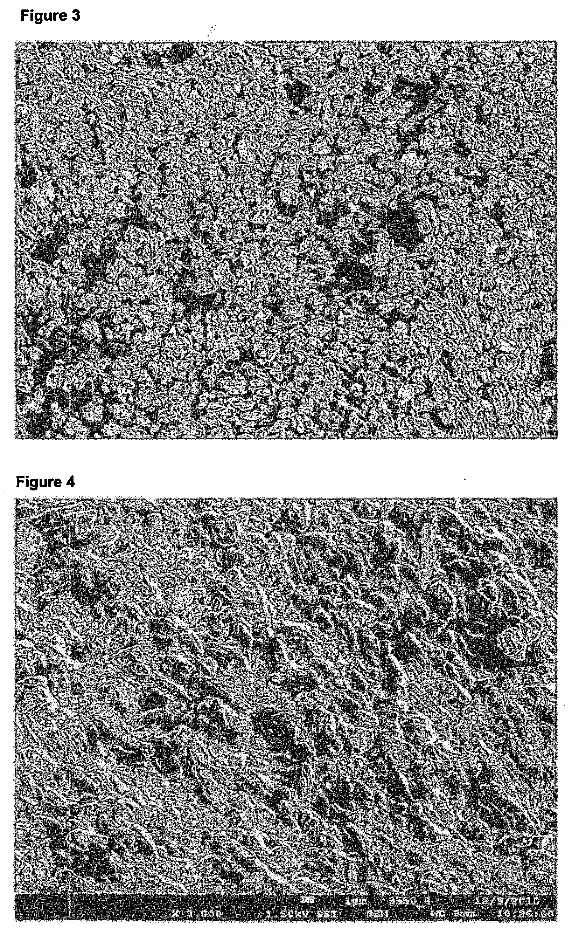

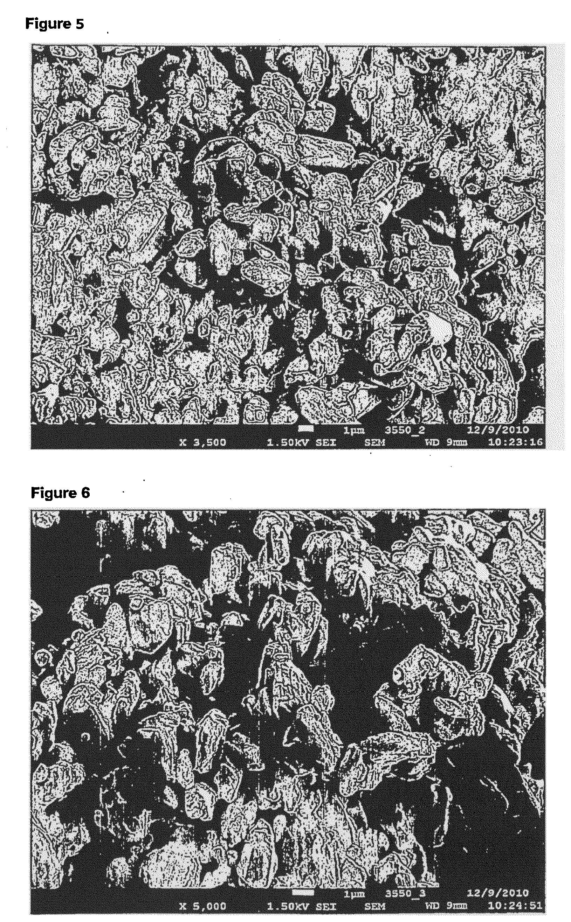

Figures 3 to 6 depict the outer surface of the API bead shown in Fig. 2, magnified by factor 1500, 300, 3500 and 5000 respectively. Figs. 3 and 4 demonstrate that the outer surface of the API bead has a large amount of cavities formed in between API primary particles, wherein these cavities provide for an advantageous porosity. In Figs. 5 and 6, the single API primary particles are clearly visible, wherein an API primary particle size of about 1 to 5 $\mu$m is derivable.

**Examples**

**Reference Example 1**

Suspension composition:

| Substance | Amount [g] |
|---|---|
| ketoprofen (particulate) | 60.00 |
| Polysorbate 80 | 5.00 |
| Sodium Chloride | 11.34 |
| Water | 35.00 |

Manufacturing procedure:

Sodium chloride and polysorbate were dissolved in water and then ketoprofen (2-(3-benzoylphenyl)-propionic acid) was dispersed in the resulting solution to form a homogenous suspension. A 50 ml milling vessel was loaded with 8 ml of zirconia milling beads having a diameter of 0.6 to 0.8 mm. Next, the milling vessel was loaded with 12 ml of ketoprofen suspension having the above mentioned composition. The milling vessel was placed in a ball mill and rotated, i.e. the suspension was milled, wherein revolution speed was 250 round per minute (RPM) and milling time was 30 minutes.

The resulting product was a suspension wherein particle size of ketoprofen was decreased compared to the originally applied ketoprofen particels.

**Reference Example 2**

Suspension composition:

| Substance | Amount [g] |
|---|---|
| ketoprofen (particulate) | 35.00 |
| Polysorbate 80 | 2.75 |
| Water | 62.25 |

Manufacturing procedure:

Polysorbate was dissolved in water and then ketoprofen was dispersed in the resulting solution to form a homogenous suspension. A 50 ml milling vessel was loaded with 24 ml of zirconia milling beads having a diameter of 0.6 to 0.8 mm. Next, the milling vessel was loaded with 16 ml of ketoprofen suspension having the above mentioned composition.

The milling vessel was placed in a ball mill and rotated, i.e. the suspension was milled, wherein revolution speed was 400 RPM and milling time was 195 minutes. The resulting product was a suspension wherein particle size of ketoprofen was decreased compared to the originally applied ketoprofen particels.

**Example 3 (according to the invention)**

Suspension composition:

| Substance | Amount [g] |
|---|---|
| ketoprofen (particulate) | 35.00 |
| Polysorbate 80 | 2.75 |
| Sodium Chloride | 10.08 |

(continued)

| Substance | Amount [g] |
|---|---|
| Water | 62.25 |

Manufacturing procedure:

Sodium chloride and polysorbate were dissolved in water and then ketoprofen was dispersed in the resulting solution to form a homogenous suspension. A 50 ml milling vessel was loaded with 24 ml of zirconia milling beads having a diameter of 0.6 to 0.8 mm. Next, the milling vessel was loaded with 16 ml of ketoprofen suspension having the above mentioned composition. The milling vessel was placed in a ball mill and rotated, i.e. the suspension was milled, wherein revolution speed was 400 RPM and milling time was 195 minutes.

The resulting product is an API preparation in the form of beads (API bead(s)) which is separate from the remaining aqueous phase.

**Example 4 (according to the invention)**

Suspension composition:

| Substance | Amount [g] |
|---|---|
| ketoprofen (particulate) | 15.00 |
| Polysorbate 80 | 1.80 |
| Sodium Chloride | 20.97 |
| Water | 83.20 |

Manufacturing procedure:

Sodium chloride and polysorbate were dissolved in water and then ketoprofen was dispersed in the resulting solution to form a homogenous suspension. A 50 ml milling vessel was loaded with 18 ml of zirconia milling beads having a diameter of 0.6 to 0.8 mm. Next, the milling vessel was loaded with 12 ml of ketoprofen suspension having the above mentioned composition. The milling vessel was placed in a ball mill and rotated, i.e. the suspension was milled, wherein revolution speed was 500 RPM and milling time was 30 minutes.

The resulting product is an API preparation in the form of beads (API bead(s)) which is separate from the remaining aqueous phase, wherein ketoprofen was layered onto the majority of the milling beads.

**Example 5 (according to the invention)**

Suspension composition:

| Substance | Amount [g] |
|---|---|
| ketoprofen (particulate) | 20.00 |
| Polysorbate 80 | 1.50 |
| Sodium Chloride | 25.43 |
| Water | 78.50 |

Manufacturing procedure:

Sodium chloride and polysorbate were dissolved in water and then ketoprofen was dispersed in the resulting solution to form a homogenous suspension. A 50 ml milling vessel was loaded with 30 ml of zirconia milling beads having a diameter of 0.6 to 0.8 mm. Next, the milling vessel was loaded with 20 ml of ketoprofen suspension having the above mentioned composition. The milling vessel was placed in a ball mill and rotated, i.e. the suspension was milled, wherein revolution speed was 300 RPM and milling time was 360 minutes.

The resulting product is an API preparation in the form of beads (API bead(s)) which is separate from the remaining aqueous phase. About 30 API beads were formed, wherein there was essentially no layering of ketoprofen on the milling beads as described for Example 4.

**Example 6 (according to the invention)**

Suspension composition:

| Substance | Amount [g] |
|---|---|
| ketoprofen (particulate) | 20.00 |
| Polysorbate 80 | 4.50 |
| Sodium Chloride | 24.46 |
| Water | 75.5 |

Manufacturing procedure:

Sodium chloride and polysorbate were dissolved in water and then ketoprofen was dispersed in the resulting solution to form a homogenous suspension. A 50 ml milling vessel was loaded with 30 ml of zirconia milling beads having a diameter of 0.6 to 0.8 mm. Next, the milling vessel was loaded with 25 ml of ketoprofen suspension having the above mentioned composition. The milling vessel was placed in a ball mill and rotated, i.e. the suspension was milled, wherein revolution speed was 300 RPM and milling time was 120 minutes.

The resulting product is an API preparation in the form of beads (API bead(s)) which is separate from the remaining aqueous phase, wherein there was essentially no layering of ketoprofen on the milling beads.

**Example 7 (according to the invention)**

Suspension composition:

| Substance | Amount [g] |
|---|---|
| ketoprofen (particulate) | 40.00 |
| Polysorbate 80 | 1.50 |
| Sodium Chloride | 18.95 |
| Water | 58.50 |

Manufacturing procedure:

Sodium chloride and polysorbate were dissolved in water and then ketoprofen was dispersed In the resulting solution to form a homogenous suspension. A 50 ml milling vessel was loaded with 30 ml of zirconia milling beads having a diameter of 0.6 to 0.8 mm. Next, the milling vessel was loaded with 25 ml of ketoprofen suspension having the above mentioned composition. The milling vessel was placed in a ball mill and rotated, i.e. the suspension was milled, wherein revolution speed was 350 RPM and milling time was 120 minutes.

The resulting product is an API preparation in the form of beads (API bead(s)) which is separate from the remaining aqueous phase, wherein there was essentially no layering of ketoprofen on the milling beads.

**Example 8 (according to the invention)**

Suspension composition:

| Substance | Amount [g] |
|---|---|
| ketoprofen (particulate) | 22.50 |
| Polysorbate 80 | 1.65 |
| Sodium Chloride | 17.75 |
| Water | 75.85 |

Manufacturing procedure:

Sodium chloride and polysorbate were dissolved in water and then ketoprofen was dispersed in the resulting solution to form a homogenous suspension. A 50 ml milling vessel was loaded with 30 ml of zirconia milling beads having a diameter of 0.6 to 0.8 mm. Next, the milling vessel was loaded with 25 ml of ketoprofen suspension having the above mentioned composition. The milling vessel was placed in a ball mill and rotated, i.e. the suspension was milled, wherein revolution speed was 325 RPM and milling time was 240 minutes.

The resulting product is an API preparation in the form of beads (API bead(s)) which is separate from the remaining aqueous phase, wherein there was essentially no layering of ketoprofen on the milling beads.

**Example 9 (according to the invention)**

The dissolution profile of API beads prepared in examples 6, 7 and 8 were determined with an USP Apparatus 2 (paddles) in 900 ml of phosphate buffer of pH 5.7. In this dissolution testing under sink conditions, the API composition in the form of beads surprisingly exhibited different dissolution behavior. The beads obtained in Example 7 exhibit a relatively quick (immediate) release profile, the beads obtained in Example 8 exhibit a markedly reduced (i.e. prolonged) dissolution profile, and the beads obtained in Example 6 exhibit a dissolution profile in between the quick and reduced dissolution profile.

**Explanatory notes to the Examples**

In Examples 1-8, the average original particle size of API applied as the starting material, that is particulate ketoprofen, was about 15 μm.
In Examples 3-8, API beads having a porosity of about 10% were obtained.

**Claims**

1. A process for producing an active pharmaceutical ingredient (API) preparation in the form of beads, wherein the process comprises:

a) providing an aqueous suspension comprising an API in the form of undissolved particles, a stabilizing agent, an inorganic salt and water,
b) subjecting an aqueous suspension defined in a) to ball milling, wherein the average original particle size of the API provided in a) is increased by aggregation, the aggregated API preparation in the form of beads having an average diameter of at least 0.1 mm,

wherein the average original particle size of the API provided in a) is 5 μm to 1 mm, and wherein in b) the revolution speed of ball milling is set within a range of 300 to 500 min-1.

2. The process according to claim 1, **characterized by** either one or a combination of the following features i) to x):

i) a ratio of average original particle size of the API provided in a) to diameter of the API beads obtained in b) is 1/1000 to 1/10, preferably 2/1000 to 8/100;
ii) the API provided in a) *per se* has a solubility in water of less than 1 g/l, preferably less than 0.6 g/l;
iii) in the aqueous suspension provided in a), the weight ratio of amount of API to amount of water (API/water) is up to 1/1, preferably 0.1/1 to 0.8/1 and more preferably 0.15/1 to 0.7/1;
iv) the total amount of API is 8 to 45 % by weight relative to total weight of the aqueous suspension provided in a), preferably 10 to 40 % by weight;
v) the average original particle size of the API provided in a) is 6 μm to 0.5 mm, preferably 7 μm to 250 μm and in particular 8 μm to 125 μm;
vi) the API preparation in the form of beads formed in b) have a diameter of 0.4 to 3 mm, preferably 0.5 to 2 mm;
vii) ball milling in b) is conducted at a loading with milling beads at a ratio of bulk volume of milling beads to volume of milling vessel of 0.2/1 to 0.75/1, preferably 0.22/1 to 0.6/1;
viii) the density of milling balls is 3 to 17 kg/dm$^3$, preferably 3.5 to 10 kg/dm$^3$ and more preferably 5 to 8.5 kg/dm$^3$;
ix) the milling balls have a diameter of 0,05 to 5 mm, preferably 0.4 to 2 mm, more preferably 0.6 to 0.8 mm;
x) the volume ratio of milling beads to the aqueous suspension provided in a) is 0.9/1 to 2/1, preferably 1/1 to 1.6/1.

3. The process according to claim 1 or 2, wherein the stabilizing agent is a wetting agent, preferably selected from the group consisting of polyoxyethylene sorbitan esters, polyoxyethylene alkylphenyl ethers, polyoxamers, polyoxyethylene fatty acid esters, polyoxyethylene alkyl ethers, polyoxyethylene fatty acid esters, propylene glycol fatty acid esters, fatty acids and salts thereof, glyceryl fatty acid esters, sorbitan esters, quaternary ammonium compounds, alkyl sulfates and their salts, other ionic surfactants (e,g, bile salts), mono- and diglycerides, sterol and sterol derivatives, unsaturated polyglycosed glycerides, polyoxyglycerides, and mixtures thereof, more preferably the wetting agent is a polyoxyethylene sorbitan ester, even more preferably polysorbat 20-120, and in particular polysorbat 80.

4. The process according to one of claims 1 to 3, wherein the stabilizing agent is a suspending agent, preferably selected from the group consisting of cellulose derivatives, alginates, chitosan, dextrans, gelatin, polyethylene glycols, polyoxyethylene- and polyoxypropylene ethers, polyvinylpyrrolidone, polyvinyl alcohol, and mixtures thereof, preferably a cellulose derivative selected from the group consisting of methyl cellulose, sodium carboxymethyl cellulose and hydroxypropyl methyl cellulose.

5. The process according to claim 3 or 4, wherein said wetting agent(s) and said suspending agent(s) are used alone or in combination with each other as the stabilizing agent, preferably the total amount of wetting agent(s) is 0.1 to 15 % by weight relative to total weight of the aqueous suspension provided in a) and/or the total amount of suspending agent(s) is 0.1 to 15 % by weight relative to total weight of the aqueous suspension provided in a) of claim 1, more preferably the total amount of wetting agent(s) is 0.8 to 10 % by weight relative to total weight of the aqueous suspension provided in a) and/or the total amount of suspending agent(s) is 0.8 to 10 % by weight relative to total weight of the aqueous suspension provided in a) of claim 1, and in particular the stabilizing agent is a wetting agent in the total amount of 1 to 6 % by weight relative to total weight of the aqueous suspension provided in a) of claim 1.

6. The process according to any one of claims 1 to 5, **characterized by** either one or a combination or the following features (a) to (h):

    (a) the inorganic salt is selected from the group consisting of sodium, potassium, magnesium and calcium salts of either monoatomic or polyatomic counter ions, preferably the inorganic salt is sodium chloride;
    (b) the total amount of inorganic salt(s) is 1 to 30 % by weight relative to total weight of the aqueous suspension provided in a) of claim 1, preferably 4 to 25 % by weight and more preferably 8 to 22 % by weight;
    (c) the aqueous suspension provided in a) further comprises auxiliary agent(s) or pharmaceutically acceptable excipients, preferably selected from the groups consisting of buffering agents, defoaming agents and preservatives;
    (d) the total amount of auxiliary agent(s) is 0 to 10 % by weight relative to total weight of the aqueous suspension provided in a) of claims 1, preferably 0.01 to 5 % by weight;
    (e) the API provided in a) has at least one characteristic selected from the group consisting of:

        - at least one polar group is present in the API molecule, preferably a keto and/or carboxylic acid group, more preferably a keto and a carboxylic acid group;

        - a molecular weight of 150 to 1000 g/mol, preferably 180 to 500 g/mol, more preferably 220 to 400 g/mol and in particular 240 to 300 g/mol;
        - a melting point of 60-150 °C, preferably 80-110 °C, more preferably 90-100;

    (f) the API belongs to the class of APIs to be orally administered, preferably non-opioid analgesic, more preferably ketoprofen;
    (g) the beads obtained from the ball milling are substantially spherical, preferably having a sphericity of at least 0.5, more preferably 0.7, and in particular 0.8;
    (h) the beads obtained from the ball milling have a porosity of 1 to 30%, preferably 5 to 20%, more preferably 7 to 15% and in particular 8 to 12%.

7. The process according to any one of claims 1 to 6, **characterized by** either one or a combination of the following features (i) to (v):

    (i) in b) of claim 1, the milling time is set within a range of 20 to 400 min, preferably 30 to 360 min, more preferably 100 to 250 min;
    (ii) in b) of claim 1, the volume ratio of total load volume of aqueous suspension of a) to volume of the milling vessel is in a range of 0.2/1 to 0.6/1, preferably 0.22/1 to 0.5/1;
    (iii) the milling ball material has a Mohs hardness of 5 to 10, preferably 6 to 9.5 and more preferably 8 to 9;
    (iv) the milling ball material is selected from the group consisting of hard porcelain, steatite, $Al_2O_3$, $ZrO_2$, steel, hard metal, preferably the milling ball material is selected from the group consisting of $Al_2O_3$, $ZrO_2$, steel, more preferably the milling ball material is $ZrO_2$;
    (v) the surface of the milling ball is coated or uncoated, preferably uncoated.

8. An active pharmaceutical ingredient (API) preparation in the form of beads based on an API having a solubility in water of less than 1 g/l wherein:

    (i) the beads of the API preparation are formed by ball milling particulate API to obtain an API preparation in the form of beads having an average diameter of at least 0.1 mm; and
    (ii) API primary particles, which are comprised in the API preparation in the form of beads, have an average diameter of less than 20 $\mu$m,

wherein the bead porosity is 1 to 30%, and wherein the API preparation is in the form of spherical beads having a sphericity of at least 0.5.

9. The API preparation according to claim 8, **charac-**

**terized by** either one or a combination of the following features a) to f):

a) ball milling as defined in (i) is accomplished by a ball milling process according to any one of claims 1 to 7;
b) the porosity is 5 to 20%, preferably 7 to 15% and in particular 8 to 12%;
c) the API preparation in the form of beads comprises the API in an amount of at least 90% by weight relative to the weight of the composition in the form of beads, preferably 93.00-99.99% by weight, more preferably 96.00-99.90% by weight;
d) the API preparation in the form of beads is prepared by a ball milling process, preferably a ball milling process according to any one of claims (1) to (7);
e) the API comprised in said bead has characteristics selected from the group consisting of:

- at least one polar group is present in the API molecule, preferably a keto and/or carboxylic acid group, more preferably a keto and a carboxylic acid group;
- a molecular weight of 150 to 1000 g/mol, preferably 180 to 500 g/mol, more preferably 220 to 400 g/mol and in particular 240 to 300 g/mol;
- a melting point of 60-150 °C, preferably 80-110 °C, more preferably 90-100°C;
- a solubility in water less or equal to 0.6 g/l, more preferably less or equal to 0.1 mg/ml, and in particular less or equal to 0.01 mg/ml;

f) the API preparation in the form of beads has a sphericity of at least 0.7, and in particular 0.8.

**10.** A pharmaceutical composition comprising the API preparation according to claim 8 or 9 or consisting only of the API preparation according to claims 8 or 9.

**11.** The pharmaceutical composition according to claim 10, wherein the composition is in a form selected from pellets, granules or compressed tablets, and/or the composition is in a form filled in sachets or in capsules, preferably the pharmaceutical composition is in form of a capsule filled with pellets.

**Patentansprüche**

**1.** Verfahren zur Herstellung einer Zubereitung eines pharmazeutischen Wirkstoffes (API) in der Form von Kügelchen, wobei das Verfahren umfasst:

a) Bereitstellen einer wässrigen Suspension, die einen API in der Form von ungelösten Partikeln, ein Stabilisierungsmittel, ein anorganisches Salz und Wasser umfasst,
b) Unterziehen der in a) definierten wässrigen Suspension einem Kugelmahlen, wobei die durchschnittliche ursprüngliche Partikelgröße des API, der in a) bereitgestellt wurde, durch Aggregation erhöht wird, wobei die aggregierte API-Zubereitung in der Form von Kügelchen einen durchschnittlichen Durchmesser von mindestens 0,1 mm besitzt,

wobei die in a) bereitgestellte durchschnittliche ursprüngliche Partikelgröße des API 5 $\mu$m bis 1 mm beträgt, und wobei in b) die Drehzahl des Kugelmahlens in einem Bereich von 300 bis 500 min$^{-1}$ festgelegt ist.

**2.** Verfahren gemäß Anspruch 1, **gekennzeichnet durch** entweder eines oder eine Kombination der folgenden Merkmale i) bis x):

i) ein Verhältnis von durchschnittlicher ursprünglicher Partikelgröße des API, wie er in a) bereitgestellt wurde, zum Durchmesser der API-Kügelchen, wie sie in b) erhalten wurden, beträgt 1/1000 bis 1/10, vorzugsweise 2/1000 bis 8/100;
ii) der in a) bereitgestellte API hat *per se* eine Löslichkeit in Wasser von weniger als 1 g/l, vorzugsweise weniger als 0,6 g/l;
iii) in der in a) bereitgestellten wässrigen Suspension beträgt das Gewichtsverhältnis der Menge von API zur Menge von Wasser (API/Wasser) bis zu 1/1, vorzugsweise 0,1/1 bis 0,8/1 und weiter vorzugsweise 0,15/1 bis 0,7/1;
iv) die Gesamtmenge von API ist 8 bis 45 Gewichts-% bezogen auf das Gesamtgewicht der in a) bereitgestellten wässrigen Lösung, vorzugsweise 10 bis 40 Gewichts-%;
v) die durchschnittliche ursprüngliche Partikelgröße des in a) bereitgestellten API ist 6 $\mu$m bis 0,5 mm, vorzugsweise 7 $\mu$m bis 250 $\mu$m und insbesondere 8 $\mu$m bis 125 $\mu$m;
vi) die in b) ausgebildete API-Zubereitung in der Form von Kügelchen haben einen Durchmesser von 0,4 bis 3 mm, vorzugsweise 0,5 bis 2 mm;
vii) das Kugelmahlen in b) wird bei einer Beladung mit Mahlkügelchen bei einem Verhältnis des Bulkvolumens von Mahlkügelchen zum Volumen des Mühlengefäßes von 0,2/1 bis 0,75/1 durchgeführt, vorzugsweise bei 0,22/1 bis 0,6/1;
viii) die Dichte der Mahlkügelchen beträgt 3,0 bis 17 kg/dm$^3$, vorzugsweise 3,5 bis 10,0 kg/dm$^3$, und weiter vorzugsweise 5,0 bis 8,5 kg/dm$^3$;
ix) die Mahlkügelchen haben einen Durchmesser von 0,05 bis 5,0 mm, vorzugsweise 0,4 bis 2,0 mm, weiter vorzugsweise 0,6 bis 0,8 mm;

x) das Volumenverhältnis der in a) bereitgestellten Mahlkügelchen zur wässrigen Suspension beträgt 0,9/1 bis 2/1, vorzugsweise 1/1 bis 1,6/1.

3.  Verfahren gemäß Anspruch 1 oder 2, wobei das Stabilisierungsmittel ein Benetzungsmittel ist, vorzugsweise ausgewählt aus der Gruppe bestehend aus Polyoxyethylensorbitanestern, Polyoxyethylenalkyl-Phenylethern, Polyoxameren, Polyoxyethylen-Fettsäureestern, Polyoxyethylen-Alkylethern, Polyoxyethylen-Fettsäureestern, Propylenglycol-Fettsäureestern, Fettsäuren und Salzen davon, Glyceryl-Fettsäureestern, Sorbitanestern, quaternären AmmoniumVerbindungen, Alkylsulfaten und ihren Salzen, anderen ionischen oberflächenaktiven Stoffe (z.B. Gallensalzen), Mono- und Diglyceriden, Sterol und Sterolderivaten, ungesättigten polyglycosidischen Glyceriden, Polyoxyglyceriden, und Mischungen davon, wobei das Benetzungsmittel weiter bevorzugt ein Polyoxyethylen-Sorbitanester ist, noch weiter bevorzugt Polysorbat 20-120 und insbesondere Polysorbat 80 ist.

4.  Verfahren gemäß einem der Ansprüche 1 bis 3, wobei das Stabilisierungsmittel ein suspendierendes Mittel ist, vorzugsweise ausgewählt aus der Gruppe bestehend aus Zellulosederivativen, Alginaten, Chitosan, Dextranen, Gelatine, Polyethylenglycolen, Polyoxyethylen- und Polyoxypropylenethern, Polyvinylpyrrolidon, Polyvinylalkohol, und Mischungen davon, vorzugsweise ein Cellulosederivat, ausgewählt aus der Gruppe bestehend aus Methylcellulose, Natriumcarboxymethylcellulose und Hydroxypropylmethylcellulose.

5.  Verfahren gemäß Anspruch 3 oder 4, wobei das/die Benetzungsmittel und das/die suspendierende(n) Mittel alleine oder in Kombination miteinander als das Stabilisierungsmittel verwendet werden, wobei die Gesamtmenge von Benetzungsmittel(n) vorzugsweise 0,1 bis 15 Gewichts-% bezogen auf das Gesamtgewicht der in a) bereitgestellten wässrigen Suspension ist und/oder der Gesamtmenge von suspendierendem/n Mittel(n) 0,1 bis 15 Gewichts-% bezogen auf das Gesamtgewicht der in a) von Anspruch 1 bereitgestellten wässrigen Lösung ist, weiter vorzugsweise die Gesamtmenge von Benetzungsmittel(n) 0,8 bis 10 Gewichts-% bezogen auf das Gesamtgewicht der in a) bereitgestellten wässrigen Suspension ist, und/oder die Gesamtmenge von suspendierendem/n Mittel(n) 0,8 bis 10 Gewichts-% bezogen auf das Gesamtgewicht der in a) von Anspruch 1 bereitgestellten wässrigen Suspension, und insbesondere das Stabilisierungsmittel ein Benetzungsmttel in der Gesamtmenge von 1 bis 6 Gewichts-% bezogen auf das Gesamtgewicht der in a) von Anspruch 1 bereitgestellten wässrigen Suspension, ist.

6.  Verfahren gemäß einem der Ansprüche 1 bis 5, **gekennzeichnet durch** entweder eines oder eine Kombination der folgenden Merkmale (a) bis (h):

(a) das anorganische Salz ist ausgewählt aus der Gruppe bestehend aus Natrium, Kalium, Magnesium und Calciumsalzen von entweder einatomigen oder mehratomigen Gegenionen, wobei vorzugsweise das anorganische Salz Natriumchlorid ist;

(b) die Gesamtmenge des/der anorganischen Salze(s) ist 1 bis 30 Gewichts-% bezogen auf das Gesamtgewicht der wässrigen Suspension, wie sie in a) von Anspruch 1 bereitgestellt wurde, vorzugsweise 4 bis 25 Gewichts-% und weiter vorzugsweise 8 bis 22 Gewicht-%;

(c) die wässrige Lösung, wie sie in a) bereitgestellt wurde, weist weiterhin (ein) Hilfsmittel oder pharmazeutisch akzeptable Hilfsstoffe auf, vorzugsweise ausgewählt aus den Gruppen bestehend aus Pufferagenzien, Entschäumungsmitteln und Konservierungsstoffen;

(d) die Gesamtmenge von Hilfsmittel(n) beträgt 0 bis 10 Gewichts-% bezogen auf das Gesamtgewicht der in a) von Anspruch 1 bereitgestellten wässrigen Suspension, vorzugsweise 0,01 bis 5 Gewichts-%;

(e) der API, wie er in a) bereitgestellt wurde, hat mindestens ein Merkmal, ausgewählt aus der Gruppe bestehend aus:

- mindestens eine polare Gruppe ist in dem API-Molekül vorhanden, vorzugsweise eine Keto- und/oder Carboxylsäuregruppe, weiter vorzugsweise eine Keto- und eine Carboxylsäuregruppe;
- ein Molekulargewicht von 150 bis 1000 g/mol, vorzugsweise 180 bis 500 g/mol, weiter vorzugsweise 220 bis 400 g/mol und insbesondere 240 bis 300 g/mol;
- ein Schmelzpunkt von 60-150°C, vorzugsweise 80-110°C, weiter vorzugsweise 90-100°C;

(f) der API gehört zur Klasse von den APIs, welche oral verabreicht werden, vorzugsweise nicht-opioides Analgetikum, weiter vorzugsweise Ketoprofen;

(g) die Kügelchen, welche vom Kugelmahlen erhalten werden, sind im Wesentlichen kugelförmig, weiter vorzugsweise haben sie eine Sphärizität von mindestens 0,5, weiter vorzugsweise 0,7, und insbesondere 0,8;

(h) die Kügelchen, welche vom Kugelmahlen erhalten werden, haben eine Porosität von 1 bis 30%, vorzugsweise 5 bis 20%, weiter vorzugsweise 7 bis 15% und insbesondere 8 bis 12%.

**7.** Verfahren gemäß einem der Ansprüche 1 bis 6, **gekennzeichnet durch** entweder eines oder eine Kombination der folgenden Merkmale (i) bis (v):

> (i) in b) von Anspruch 1 ist die Mahlzeit in einem Bereich von 20 bis 400 min, vorzugsweise 30 bis 360 min, weiter vorzugsweise 100 bis 250 min festgelegt;
> (ii) in b) von Anspruch 1 liegt das Volumenverhältnis des Gesamtbeladungsvolumens der wässrigen Suspension von a) zum Volumen des Mahlgefäßes in einem Bereich von 0,2/1 bis 0,6/1, vorzugsweise 0,22/1 bis 0,5/1;
> (iii) das Mahlkugelmaterial hat eine Mohs-Härte von 5 bis 10, vorzugsweise 6 bis 9,5 und weiter vorzugsweise 8 bis 9;
> (iv) das Mahlkugelmaterial ist ausgewählt aus der Gruppe bestehend aus hartem Porzellan, Steatit, $Al_2O_3$, $ZrO_2$, Stahl, Hartmetall, vorzugsweise ist das Mahlkugelmaterial ausgewählt aus der Gruppe bestehend aus $Al_2O_3$, $ZrO_2$, Stahl, weiter vorzugsweise ist das Mahlkugelmaterial $ZrO_2$;
> (v) die Oberfläche der Mahlkugel ist beschichtet oder unbeschichtet, vorzugsweise unbeschichtet.

**8.** Zubereitung von pharmazeutischem Wirkstoff (API) in der Form von Kügelchen basierend auf einem API, welcher eine Löslichkeit in Wasser von weniger als 1 g/l besitzt, wobei:

> (i) die Kügelchen der API-Zubereitung durch Kugelmahlen von partikulärem API gebildet wurden, um eine API-Zubereitung in der Form von Kügelchen zu erhalten, welche einen durchschnittlichen Durchmesser von mindestens 0,1 mm besitzen; und
> (ii) API-Primärpartikel, welche in der API-Zubereitung in der Form von Kügelchen enthalten sind, einen durchschnittlichen Durchmesser von weniger als 20 μm haben,

wobei die Kügelchenporosität 1 bis 30% beträgt, und wobei die API-Zubereitung in der Form von kugelförmigen Kügelchen vorliegt, welche eine Sphärizität von mindestens 0,5 besitzen.

**9.** API-Zubereitung gemäß Anspruch 8, **gekennzeichnet durch** entweder eines oder eine Kombination der folgenden Merkmale a) bis f):

> a) wie in (i) definiertes Kugelmahlen wird **durch** ein Kugelmühleverfahren gemäß einem der Ansprüche 1 bis 7 vorgenommen;
> b) die Porosität beträgt 5 bis 20%, vorzugsweise 7 bis 15% und insbesondere 8 bis 12%;
> c) die API-Zubereitung in der Form von Kügelchen umfasst den API in einer Menge von mindestens 90 Gewichts-% bezogen auf das Gewicht der Zusammensetzung in der Form von Kügelchen, vorzugsweise 93,00-99,99 Gewichts-%, weiter vorzugsweise 96,00-99,90 Gewichts-%;
> d) die API-Zubereitung in der Form von Kügelchen wurde durch ein Kugelmahlverfahren hergestellt, vorzugsweise durch ein Kugelmahlverfahren gemäß einem der Ansprüche (1) bis (7);
> e) der im Kügelchen enthaltene API hat Merkmale, welche ausgewählt sind aus der Gruppe bestehend aus:

>> - mindestens eine polare Gruppe ist im API-Molekül vorhanden, vorzugsweise eine Keto- und/oder Carboxylsäuregruppe, weiter vorzugsweise eine Keto- und eine Carboxylsäuregruppe;
>> - ein Molekulargewicht von 150 bis 1000 g/mol, vorzugsweise 180 bis 500 g/mol, weiter vorzugsweise 220 bis 400 g/mol und insbesondere 240 bis 300 g/mol;
>> - ein Schmelzpunkt von 60-150°C, vorzugsweise 80-110°C, weiter vorzugsweise 90-100°C;
>> - eine Löslichkeit in Wasser von weniger oder gleich 0,6 g/l, weiter vorzugsweise weniger oder gleich 0,1 mg/ml, und insbesondere weniger oder gleich 0,01 mg/ml;

> f) die API-Zubereitung in der Form von Kügelchen hat eine Sphärizität von mindestens 0,7 und insbesondere 0,8.

**10.** Pharmazeutische Zusammensetzung, umfassend die API-Zubereitung gemäß Anspruch 8 oder 9 oder bestehend nur aus der API-Zubereitung gemäß Anspruch 8 oder 9.

**11.** Pharmazeutische Zusammensetzung gemäß Anspruch 10, wobei die Zusammensetzung in einer Form vorliegt, die aus Pellets, Granulaten oder verpressten Tabletten ausgewählt ist, und/oder die Zusammensetzung liegt in einer Form vor, dass sie in Beutel oder Kapseln eingefüllt ist, vorzugsweise liegt die pharmazeutische Zusammensetzung in einer Form von einer Kapsel vor, welche mit Pellets gefüllt ist.

## Revendications

**1.** Procédé de production d'une préparation d'un ingrédient pharmaceutique actif (IPA) sous forme de billes, dans lequel le procédé comprend :

> a) la fourniture d'une suspension aqueuse comprenant un IPA sous forme de particules non dissoutes, un agent stabilisant, un sel inorganique et de l'eau,

b) la soumission d'une suspension aqueuse définie en a) à un broyage à boulets, dans lequel la taille moyenne de particules d'origine de l'IPA fourni en a) est augmentée par agrégation, la préparation d'IPA agrégé sous forme de billes ayant un diamètre moyen d'au moins 0,1 mm,

dans lequel la taille moyenne de particules d'origine de l'IPA fourni en a) est de 5 μm à 1 mm, et dans lequel en b) la vitesse de révolution du broyage à boulets est réglée selon une plage de 300 à 500 min$^{-1}$.

2. Procédé selon la revendication 1, **caractérisé par** l'une ou l'autre ou une combinaison des caractéristiques i) à x) suivantes :

i) un rapport entre la taille moyenne de particules d'origine de l'IPA fourni en a) et le diamètre des billes d'IPA obtenues en b) est de 1/1 000 à 1/10, de préférence de 2/1 000 à 8/100 ;

ii) l'IPA fourni en a) en soi a une solubilité dans l'eau inférieure à 1 g/L, de préférence inférieure à 0,6 g/L ;

iii) dans la suspension aqueuse fournie en a), le rapport en poids entre la quantité d'IPA et la quantité d'eau (IPA/eau) va jusqu'à 1/1, de préférence de 0,1/1 à 0,8/1 et de manière davantage préférée de 0,15/1 à 0,7/1 ;

iv) la quantité totale d'IPA est de 8 à 45% en poids par rapport au poids total de la suspension aqueuse fournie en a), de préférence de 10 à 40% en poids ;

v) la taille moyenne de particule d'origine de l'IPA fourni en a) est de 6 μm à 0,5 mm, de préférence de 7 μm à 250 μm et notamment de 8 μm à 125 μm ;

vi) la préparation d'IPA sous forme de billes formées en b) a un diamètre de 0,4 à 3 mm, de préférence de 0,5 à 2 mm ;

vii) le broyage à boulets en b) est mené à un chargement en billes de broyage à un rapport entre le volume en vrac de billes de broyage et le volume de la cuve de broyage de 0,2/1 à 0,75/1, de préférence de 0,22/1 à 0,6/1 ;

viii) la masse volumique des boulets de broyage est de 3 à 17 kg/dm$^3$, de préférence de 3,5 à 10 kg/dm$^3$ et de manière davantage préférée de 5 à 8,5 kg/dm$^3$ ;

ix) les boulets de broyage ont un diamètre de 0,05 à 5 mm, de préférence de 0,4 à 2 mm, de manière davantage préférée de 0,6 à 0,8 mm ;

x) le rapport en volume entre les billes de broyage et la suspension aqueuse fournie en a) est de 0,9/1 à 2/1, de préférence de 1/1 à 1,6/1.

3. Procédé selon la revendication 1 ou 2, dans lequel l'agent stabilisant est un agent mouillant, de préférence choisi dans le groupe constitué par les esters de poly(oxyéthylène sorbitane), les poly(alkylphényléthers d'oxyéthylène), les poly(oxamères), les esters d'acide gras de poly(oxyéthylène), les poly(alkyléthers d'oxyéthylène), les esters d'acide gras de poly(oxyéthylène), les esters d'acide gras de propylène glycol, les acides gras et leurs sels, les esters d'acide gras de glycéryle, les esters de sorbitane, les composés ammonium quaternaire, 1 les alkylsulfates et leurs sels, d'autres tensioactifs ioniques (par exemple, 1 des sels biliaires), 1 les mono et diglycérides, un stérol et des dérivés de stérol, des glycérides polyglycosés insaturés, 1 des poly(oxyglycérides), et leurs mélanges, de manière davantage préférée l'agent mouillant est un ester de poly(oxyéthylène sorbitane), 1 et de manière encore davantage préférée le polysorbat 20-120, et notamment le polysorbat 80.

4. Procédé selon l'une des revendications 1 à 3, dans lequel l'agent stabilisant est un agent de suspension, de préférence choisi dans le groupe constitué par les dérivés de cellulose, les alginates, le chitosane, les dextranes, la gélatine, les poly(éthylène glycols), les poly(oxyéthylènes) et poly(éthers d'oxypropylène), la poly(vinylpyrrolidone), le poly(alcool vinylique) et leurs mélanges, de préférence un dérivé de cellulose choisi dans le groupe constitué par la méthylcellulose, la carboxyméthylcellulose sodique et l'hydroxypropylméthylcellulose.

5. Procédé selon la revendication 3 ou 4, dans lequel ledit ou lesdits agents mouillants et ledit ou lesdits agents de suspension sont utilisés seuls ou en combinaison les uns avec les autres en tant qu'agent stabilisant, de préférence la quantité totale d'agent(s) mouillant (s) est de 0,1 à 15% en poids par rapport au poids total de la suspension aqueuse fournie en a) et/ou la quantité totale d'agent(s) de suspension est de 0,1 à 15% en poids par rapport au poids total de la suspension aqueuse fournie en a) de la revendication 1, de manière davantage préférée la quantité totale d'agent(s) mouillant(s) est de 0,8 à 10% en poids par rapport au poids total de la suspension aqueuse fournie en a) et/ou la quantité totale d'agent(s) de suspension est de 0,8 à 10% en poids par rapport au poids total de la suspension aqueuse fournie en a) de la revendication 1, et notamment l'agent stabilisant est un agent mouillant dans la quantité totale de 1 à 6% en poids par rapport au poids total de la suspension aqueuse fournie en a) de la revendication 1.

6. Procédé selon l'une quelconque des revendications 1 à 5, **caractérisé par** l'une ou l'autre ou une combinaison des caractéristiques (a) à (h) suivantes :

(a) le sel inorganique est choisi dans le groupe constitué par les sels de sodium, de potassium, de magnésium et de calcium de contre-ions monoatomiques ou polyatomiques, de préférence, le sel inorganique est le chlorure de sodium ;
(b) la quantité totale de sel(s) inorganique(s) est de 1 à 30% en poids par rapport au poids total de la suspension aqueuse fournie en a) de la revendication 1, de préférence, de 4 à 25% en poids et, de manière davantage préférée, de 8 à 22% en poids ;
(c) la suspension aqueuse fournie en a) comprend en outre un ou des agents auxiliaires ou des excipients pharmaceutiquement acceptables, de préférence, choisis dans les groupes constitués par les agents tampons, les agents antimousses et les conservateurs ;
(d) la quantité totale d'agent(s) auxiliaire(s) est de 0 à 10% en poids par rapport au poids total de la suspension aqueuse fournie en a) de la revendication 1, de préférence, de 0,01 à 5% en poids ;
(e) l'IPA fourni en a) possède au moins une caractéristique choisie dans le groupe constitué par :

- au moins un groupe polaire est présent dans la molécule d'IPA, de préférence, une cétone et/ou un groupe acide carboxylique, de manière davantage préférée, une cétone et un groupe acide carboxylique ;
- une masse moléculaire de 150 à 1 000 g/mol, de préférence 180 à 500 g/mol, de manière davantage préférée, 220 à 400 g/mol, et notamment 240 à 300 g/mol ;
- un point de fusion de 60 à 150° C, de préférence 80 à 110° C, de manière davantage préférée 90 à 100 ;

(f) l'IPA appartient à la classe des IPA à administrer par voie orale, de préférence un analgésique non opioïde, de manière davantage préférée, le cétoprofène ;
(g) les billes obtenues par le broyage à boulets sont sensiblement sphériques, ayant de préférence une sphéricité d'au moins 0,5, de manière davantage préférée, 0,7, et notamment 0,8 ;
(h) les billes obtenues par le broyage à boulets ont une porosité de 1 à 30%, de préférence, 5 à 20%, de manière davantage préférée, 7 à 15%, et notamment 8 à 12%.

7. Procédé selon l'une quelconque des revendications 1 à 6, **caractérisé par** l'une ou l'autre ou une combinaison des caractéristiques (i) à (v) suivantes :

(i) en b) de la revendication 1, le temps de broyage est réglé dans une plage de 20 à 400 min, de préférence, 30 à 360 min, de manière davantage préférée, 100 à 250 min ;
(ii) en b) de la revendication 1, le rapport en volume entre le volume de charge total de la suspension aqueuse de a) et le volume de la cuve de broyage est dans une plage de 0,2/1 à 0,6/1, de préférence, de 0,22/1 à 0,5/1 ;
(iii) le matériau de boulets de broyage a une dureté Mohs de 5 à 10, de préférence 6 à 9,5 et de manière davantage préférée 8 à 9 ;
(iv) le matériau de boulets de broyage est choisi dans le groupe constitué par la porcelaine dure, la stéatite, $Al_2O_3$, $ZrO_2$ l'acier, le métal dur, de préférence, le matériau de boulets de broyage est choisi dans le groupe constitué par $Al_2O_3$, $ZrO_2$, l'acier, de manière davantage préférée, le matériau de boulets de broyage est $ZrO_2$ ;
(v) la surface du boulet de broyage est enrobée ou non enrobée, de préférence non enrobée.

8. Préparation d'ingrédients pharmaceutiques actifs (IPA) sous forme de billes à base d'un IPA ayant une solubilité dans l'eau inférieure à 1 g/L, dans laquelle :

(i) les billes de la préparation d'IPA sont formées par broyage à boulets d'IPA particulaire pour obtenir une préparation d'IPA sous forme de billes d'un diamètre moyen d'au moins 0,1 mm ; et
(ii) les particules primaires d'IPA, qui sont comprises dans la préparation d'IPA sous forme de billes, ont un diamètre moyen inférieur à 20 $\mu$m,

dans laquelle la porosité des billes est de 1 à 30%, et dans laquelle la préparation d'IPA est sous forme de billes sphériques ayant une sphéricité d'au moins 0,5.

9. Préparation d'IPA selon la revendication 8, **caractérisée par** l'une ou l'autre ou une combinaison des caractéristiques a) à f) suivantes :

a) le broyage à boulets tel que défini en (i) est accompli par un procédé de broyage à boulets selon l'une quelconque des revendications 1 à 7 ;
b) la porosité est de 5 à 20%, de préférence, de 7 à 15%, et notamment de 8 à 12% ;
c) la préparation d'IPA sous forme de billes comprend l'IPA dans une quantité d'au moins 90% en poids par rapport au poids de la composition sous forme de billes, de préférence de 93,00 à 99,99% en poids, de manière davantage préférée de 96,00 à 99,90% en poids ;
d) la préparation d'IPA sous forme de billes est préparée par un procédé de broyage à boulets, de préférence un procédé de broyage à boulets selon l'une quelconque des revendications (1) à (7) ;

e) l'IPA compris dans ladite bille a des caractéristiques choisies dans le groupe constitué par :

- au moins un groupe polaire est présent dans la molécule d'IPA, de préférence une cétone et/ou un groupe acide carboxylique, de manière davantage préférée une cétone et un groupe acide carboxylique ;
- une masse moléculaire de 150 à 1 000 g/mol, de préférence, de 180 à 500 g/mol, de manière davantage préférée, de 220 à 400 g/mol, et notamment de 240 à 300 g/mol ;
- un point de fusion de 60 à 150°C, de préférence, de 80 à 110°C, de manière davantage préférée, de 90 à 100 °C ;
- une solubilité dans l'eau inférieure ou égale à 0,6 g/L, de manière davantage préférée, inférieure ou égale à 0,1 mg/mL, et notamment inférieure ou égale à 0,01 mg/mL ;

f) la préparation d'IPA sous forme de billes a une sphéricité d'au moins 0,7, et notamment 0,8.

10. Composition pharmaceutique comprenant la préparation d'IPA selon la revendication 8 ou 9 ou constituée uniquement de la préparation d'IPA selon la revendication 8 ou 9.

11. Composition pharmaceutique selon la revendication 10, dans laquelle la composition est sous une forme choisie parmi les pastilles, les granules ou les comprimés, et/ou la composition est sous une forme contenue dans des sachets ou dans des capsules, de préférence la composition pharmaceutique est sous forme d'une capsule remplie de pastilles.

EP 2 471 513 B1

**Figure 1**

Dissolution of API beads

**Figure 2**

19

**Figure 3**

**Figure 4**

**Figure 5**

**Figure 6**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 02094223 A2
- EP 1338274 A1
- WO 9930687 A

### Non-patent literature cited in the description

- **A. RAHMAN.** *Recent Advances in Pelletization Technique for Oral Drug Delivery: A Review in Current Drug Delivery,* 2009, vol. 6, 122-129
- **L. PRABHAKARAN.** Pharmaceutical Micropellets : An Overview. *Latest Reviews,* 2009, vol. 7 (4
- **M. SALAKO.** Investigations into the deformity and tensile strength of pellets. *Int. J. Pharm.,* 1998, vol. 168, 49-57